# FASCICULE DE BREVET EUROPEEN

(11) **EP 1 289 420 B1**
(45) Date de publication et mention de la délivrance du brevet: **11.03.2009**
(21) Numéro de dépôt: 01945421.4
(22) Date de dépôt: 13.06.2001
(51) Int. Cl.: A61B 5/117

(54) **PROCEDE POUR IDENTIFIER UNE PERSONNE PARMI UNE POPULATION PAR DETECTION DE SES EMPREINTES DIGITALES**
VERFAHREN ZUR IDENTIFIZIERUNG EINER PERSON DURCH FINGERABDRUCKERKENNUNG
METHOD FOR IDENTIFYING A PERSON AMONG A POPULATION BY SENSING HIS FINGERPRINTS

(30) Priorité: 16.06.2000 FR 0007707
(43) Date de publication de la demande: 12.03.2003
(73) Titulaire: SAGEM Sécurité, 75015 Paris (FR)
(72) Inventeur: CHASTEL, Pierre, F-77780 Bourron-Marlotte (FR); FONDEUR, Jean-Christophe, F-92200 Neuilly (FR)
(74) Mandataire: Gorrée, Jean-Michel
(86) Numéro de dépôt international: PCT/FR2001/001838
(87) Numéro de publication internationale: WO 2001/095804

(56) Documents cités:
- WO-A-98/40962
- FR-A- 2 674 051

## Description

La présente invention concerne des perfectionnements apportés aux procédés pour identifier une personne parmi une population, par détection des empreintes digitales d'au moins un doigt de la main de ladite personne et comparaison de ces empreintes digitales avec des informations d'empreintes digitales de toutes les personnes de ladite population préalablement mémorisées dans un fichier.

On soulignera tout d'abord que l'invention se rapporte essentiellement à 1"'identification" d'une personne prise dans une population, c'est-à-dire à la comparaison d'éléments caractéristiques d'une personne avec des éléments caractéristiques mémorisés dans un fichier et regroupant les éléments caractéristiques de toutes les personnes de la population, et que l'invention ne concerne pas l'authentification" d'une personne, c'est-à-dire la comparaison d'éléments caractéristiques de la personne avec des éléments caractéristiques identiques détenus dans un fichier qui est interrogé ou bien que ladite personne détient en double (code secret de carte à puce par exemple).

Il est connu d'identifier une personne, avec un faible risque d'erreur, à l'aide des empreintes digitales d'au moins un de ses doigts d'au moins une de ses mains. Il existe actuellement divers types de dispositifs permettant de détecter les empreintes digitales, et les informations correspondantes, transformées sous forme de signaux électriques numérisés, peuvent être mémorisées à des fins de comparaison ultérieure et d'identification.

La comparaison des empreintes digitales détectées sur un doigt d'une personne avec les informations numérisées mémorisées dans un fichier est une opération relativement complexe et longue. L'identification, par ce seul critère, d'une personne parmi une population de plusieurs millions, voire plusieurs dizaines, voire même plusieurs centaines de millions de personnes reste certes réalisable, mais nécessiterait en pratique des moyens informatiques très importants.

Certes, une présélection effectuée selon d'autres critères pourrait être effectuée. Par exemple, une présélection facile pourrait être menée d'après le sexe des personnes ; mais une telle présélection ne procure qu'une réduction par approximativement deux des informations à traiter, ce qui est très insuffisant et ne réduirait pas de façon intéressante le coût précité des matériels nécessaires.

Les documents FR-A-2 674 051 et WO-A-98/40962 exposent la mise en oeuvre d'une présélection fondée sur la reconnaissance de la forme de la main d'un individu, puis d'une identification plus fine fondée sur la reconnaissance des empreintes digitales d'au moins un doigt d'une main de l'individu.

L'invention a essentiellement pour but de proposer une solution particulière au problème qui se pose, solution qui doit permettre de ramener le processus d'identification par les empreintes digitales à une comparaison portant sur un nombre suffisamment restreint d'informations mémorisées pour que cette comparaison puisse être menée dans des conditions de rapidité et de coût acceptables, tout en conservant un nombre de points caractéristiques suffisamment élevé pour que l'identification demeure fiable.

A ces fins, l'invention propose un procédé tel que défini dans la revendication 1.

Certes, on connaît déjà divers dispositifs appropriés pour détecter la géométrie d'une main d'un individu et délivrer un ensemble de signaux électriques correspondants qui, numérisés, peuvent ensuite être tenus en mémoire.

Toutefois, il n'a jamais, jusqu'ici, été proposé de combiner, successivement et dans l'ordre, une détection de la géométrie d'une main et une détection des empreintes digitales d'au moins un doigt d'au moins une main aux fins d'identification d'un individu.

La présélection d'un groupe de personnes à partir de la géométrie de la main permet de réduire de façon considérable le domaine exploratoire dans lequel va être mené le processus d'identification par comparaison des empreintes digitales. Le processus de comparaison de la géométrie d'une main est un processus comparatif purement dimensionnel qui peut être assuré rapidement, et donc à moindre coût, même sur une population très importante.

Pour fixer les idées, l'équipement nécessité par la mise en oeuvre du procédé conforme à l'invention peut être réduit dans une proportion de 10 à 1, voire de 20 à pas rapport à l'équipement à prévoir pour une identification par la seule détection des empreintes digitales.

On peut également avantageusement prévoir que, après la sélection du groupe des formes de main sensiblement identiques à celles de la personne à identifier et avant de comparer les empreintes digitales du doigt de la personne à identifier avec celles des personnes du susdit groupe sélectionné, on effectue une sélection supplémentaire sur la base d'au moins un autre critère de sélection, qui avantageusement est un critère biométrique, notamment par exemple sur le critère du sexe et/ou du type.

Ainsi, grâce à la présélection effectuée selon l'invention par détection de la géométrie d'une main, complétée éventuellement ensuite par des présélections supplémentaires (selon le sexe qui procure une réduction par deux, selon le type, selon la taille, ...), il est possible de parvenir à ce que l'identification par comparaison des empreintes digitales ne porte plus que sur une faible fraction de la population, ce qui peut être assuré de façon sûre, rapide et économiquement acceptable.

L'invention sera mieux comprise à la lecture de la description détaillée qui suit de certains modes de réalisation préférés donnés uniquement à titre d'exemples non limitatifs. Dans cette description on se réfère aux dessins annexés sur lesquels :
- la figure 1 est un schéma synoptique illustrant l'aspect essentiel du procédé visé par l'invention ;
- la figure 2 est un schéma synoptique illustrant un mode de réalisation préféré du procédé visé par l'invention ; et
- la figure 3 est un schéma synoptique illustrant une variante conforme à l'invention pour la mise en oeuvre d'une partie des procédés des figures 1 et 2.

Les moyens concernés par l'invention doivent permettre, aux fins d'identification d'une personne appartenant à une population très nombreuse, d'écarter une grande fraction de ladite population (par exemple 70 à 80 % de celle-ci) avant d'effectuer la démarche d'identification de la personne par comparaison des empreintes digitales d'au moins un doigt d'au moins une de ses mains avec les empreintes digitales mémorisées dans un fichier central renfermant toutes les données d'identité de l'ensemble de la population.

Il est possible d'effectuer, cette présélection par relevé de la forme (géométrie) d'au moins une main de la personne, sachant que la forme d'une main peut être identifiée par un nombre donné (par exemple une douzaine) de mesures dimensionnelles prises en des points caractéristiques de la main (par exemple longueurs des doigts, largeurs des doigts à certaines articulations, épaisseur de la main, ... ) propres à donner une image tridimensionnelle de la main. De nombreux procédés et appareils sont connus à ce sujet.

De même on connaît de nombreux procédés et appareils permettant de relever les empreintes digitales d'un doigt, cette détection s'effectuant en principe sur un ou plusieurs doigts d'une main ou des deux mains.

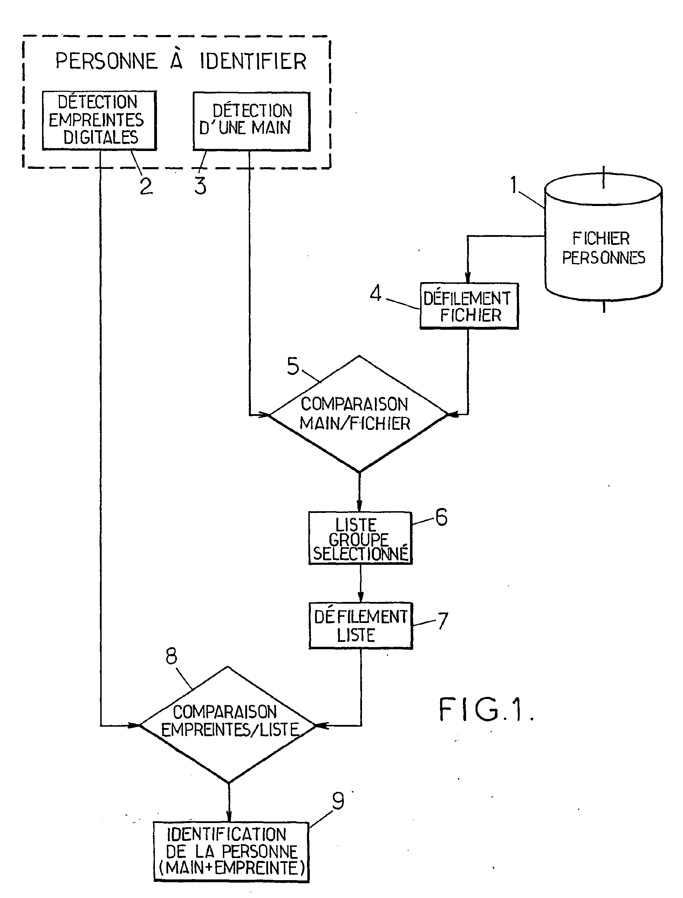

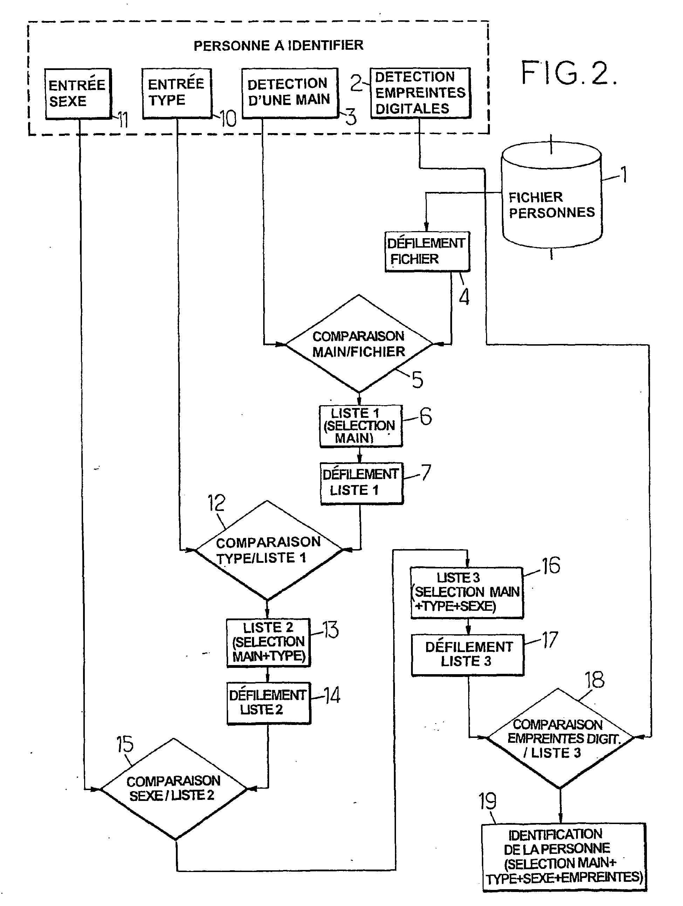

La description qui suit faite en référence aux figures 1 et 2 est destinée à aider à une meilleure compréhension de l'invention.

Dans une phase initiale de recensement, on met en mémoire dans un fichier central 1 (voir fig. 1) toutes les données d'identification de chaque personne d'une population, données d'identification qui, outre les données habituelles (nom, prénom, ...), englobent également les empreintes digitales d'au moins un doigt (par exemple index) d'au mains une main (par exemple main droite) ainsi que les données dimensionnelles relatives à la géométrie d'au moins une des mains (par exemple main droite).

Ultérieurement, pour contrôler l'identité d'une personne (contrôle d'identité de police, vote, recensement, ...), on détecte sur la personne des caractéristiques physiques d'identification, et notamment ses empreintes digitales (en 2) et la géométrie de sa main (en 3) dans les mêmes conditions que lors du recensement initial.

Dans une première étape, en commence par traiter l'information de géométrie de la main détectée sur la personne à identifier. A cette fin, par défilement en 4 des données de géométrie de main détenues dans le fichier central 1 pour toutes les personnes de la population, et par comparaison en 5 avec celles détectées sur la personne à identifier, on constitue en 6 une liste du groupe des personnes de la population qui possèdent une géométrie de main sensiblement analogue à celle de la personne à identifier.

Comme indiqué plus haut, on a ainsi restreint dans des proportions très importantes le domaine à explorer, alors que la sélection d'après la géométrie de la main, qui porte uniquement sur des comparaisons dimensionnelles, est aisée et rapide à traiter avec les moyens informatiques puissants actuellement disponibles.

On procède ensuite, en faisant défiler en 7 la liste des groupes des personnes sélectionnées en 6, par comparaison individuelle en 8 des empreintes digitales détectées en 2 sur la personne à identifier avec celles de chacune des personnes du groupe sélectionné en 6.

On obtient finalement en 9 le résultat d'identification de la personne à identifier.

Dans ces conditions, s'agissant d'une population nombreuse (par exemple celle d'un pays), les moyens proposés par l'invention permettent de faire porter l'identification comparative par les empreintes digitales, complexe et longue, sur un groupe réduit de personnes et évite d'avoir à la faire porter sur l'ensemble de la population (qui peut atteindre par exemple plusieurs dizaines de millions de personnes).

On notera également que, bien que ce ne soit pas là le but essentiel, on accroît incidemment la fiabilité de l'identification de la personne à identifier du fait que l'on met en oeuvre non pas un seul critère d'identification (empreintes digitales), mais deux critères (géométrie de la main et empreintes digitales). Certes le degré de fiabilité de l'identification par les empreintes digitales est très élevé. Toutefois, l'identification qui est assurée ici est conduite par voie informatique à partir des caractéristiques des empreintes digitales : un éventuel risque d'erreur dû à la détection de ces caractéristiques et au traitement informatisé peut être compensé par la mise en oeuvre du second critère de sélection. Bien que dans son principe le procédé qui vient d'être exposé en regard de la figure 1 conduise à une modalité de mise en oeuvre économiquement acceptable en pratique, il est toutefois possible de pousser plus loin la présélection sur la base de critères très simples à traiter. A cette fin, on peut faire appel à des critères tels que le type, le sexe, etc.

A titre d'exemple, à la figure 2, on a supposé que la présélection d'après la géométrie de la main de la personne à identifier était complétée par l'entrée de deux données supplémentaires constituées par son type (en 10) et son sexe (en 11).

A cet effet, à la liste (liste 1) du groupe des personnes sélectionnées dans le fichier 1 d'après la géométrie de leur main mise en défilement en 7 est comparée en 12 l'information de type, entrée en 10, de la personne à identifier.

I1 en résulte une deuxième liste (liste 2) obtenue en 13 des personnes sélectionnées parmi la population d'après le double critère de la géométrie de la main et du type.

A la liste 2 mise en défilement en 14 est ensuite comparée en 15 l'information de sexe, entrée en 11, de la personne à identifier.

I1 en résulte alors une troisième liste (liste 3) obtenue en 16 des personnes sélectionnées parmi la population d'après le triple critère de la géométrie de la main, du type et du sexe.

A la liste 3 mise en défilement en 17 peut alors être comparée en 18 l'information d'empreintes digitales, conduisant en 19 à l'identification de la personne.

La dernière phase de comparaison 18 ne porte alors que sur un nombre réduit de personnes, ce qui rend le traitement comparatif des empreintes digitales exécutable avec un matériel informatique considérablement réduit (réduction par 10 ou 20 par exemple).

Là encore, outre l'accélération du traitement d'identification et la réduction de l'investissement, en matériels, la multiplication des critères de présélection précédant l'identification par les empreintes digitales accroît sensiblement la fiabilité du procédé.

Afin d'éviter que le traitement comparatif de la géométrie de la main de la personne à identifier s'effectue par une comparaison systématique avec l'ensemble des données de la population, on crée, conformément à l'invention, pour gagner du temps, à partir du fichier principal 1, un fichier secondaire 20 dans lequel ont été créés des groupes réunissant chacun des personnes possédant sensiblement la même géométrie de main.

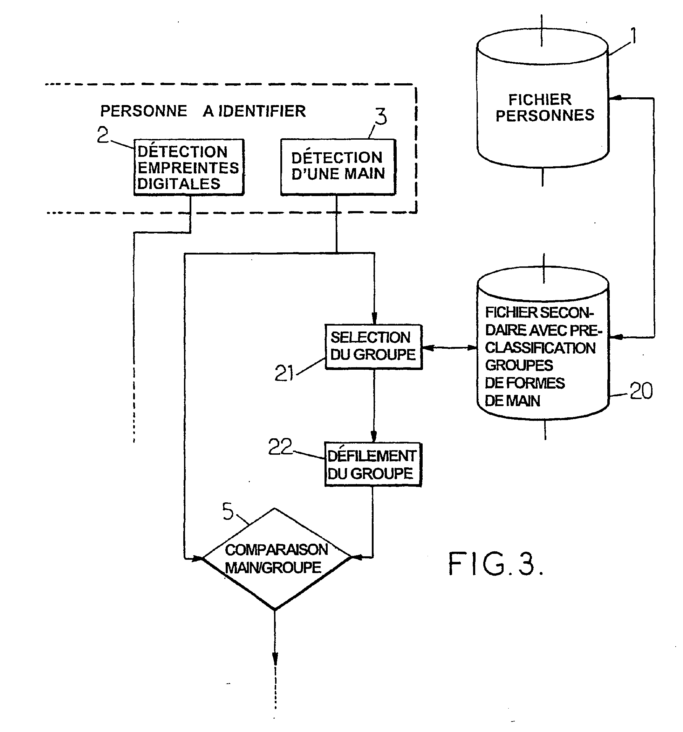

Comme illustré à la figure 3, à partir des données de géométrie de la main relevées en 3 sur la personne à identifier, on recherche en 21, dans le fichier secondaire 20, le groupe dimensionnel correspondant et c'est alors par défilement en 22 de ce seul groupe préconstitué que l'on effectue la comparaison en 5 envisagée précédemment.

Bien entendu de nombreux aménagements peuvent être apportés au procédé exposé ci-dessus sans sortir du cadre de l'invention.

## Revendications

1. Procédé pour identifier une personne appartenant à une population, par détection des empreintes digitales d'au moins un doigt de la main de ladite personne et comparaison de ces empreintes digitales détectées avec des informations d'empreintes digitales de ladite personne préalablement mémorisés dans un fichier (1), procédé dans lequel :
au cours d'une étape préalable de recensement, on détecte la forme d'au moins une main et des empreintes digitales d'au moins un doigt de la main de toutes les personnes de la population et on mémorise les informations de forme de main dans ledit fichier (1) en corrélation avec au moins les informations respectives d'empreintes digitales, on constitue, dans le fichier (1), un fichier sécondaire (20) comportant des groupes de formes de main sensiblement identiques ;
puis, au cours d'une étape ultérieur d'identification d'une personne appartenant à la population :
- on détecte (3) la forme de la main de la personne en même temps qu'on détecte (2) les empreintes digitales d'au moins un de ses doigts ;
- on sélectionne (21) dans ledit fichier sécondaire (20) un groupe constitué des formes de main sensiblement identiques à la forme de main détectée de la personne à identifier ; et
- enfin, on compare (5) les empreintes digitales détectées du doigt de la personne à identifier avec les informations d'empreintes digitales mémorisées dans ledit fichier sécondaire (20) des seules personnes du susdit groupe sélectionné.

2. Procédé selon la revendication 1, **caractérisé en ce que**, après la sélection (21) du groupe des formes de main sensiblement identiques à celles de la personne à identifier et avant de comparer les empreintes digitales du doigt de la personne à identifier avec celles des personnes du susdit groupe sélectionné, on effectue une sélection supplémentaire sur la base d'au moins un autre critère de sélection.

3. Procédé selon la revendication 2, **caractérisé en ce que** le critère de sélection supplémentaire appliqué aux personnes du groupe sélectionné de formes de main est un critère biométrique tel que sexe et/ou type.

## Claims

1. A method for identifying a person among a population, by sensing fingerprints of at least one finger of the hand of said person and comparing said sensed fingerprints with information of fingerprints of said person previously stored in a file (1), in which process:
by a previous registration step, the shape of at least one hand and fingerprints of at least one finger of the hand of all the persons of the population are sensed, and the hand shape information is stored in said file (1) in correlation with at least the respective fingerprint information, and in the file (1) a secondary file (20) is created which includes groups of substantially identical hand shapes;
then, by a later step of identification of one person belonging to the population:
- the shape of the hand of the person is sensed (3) at the same time as the fingerprints of at least one finger thereof are sensed (2);
- a group consisting of the hand shapes substantially identical to the sensed hand shape of the person to be identified is selected (21) from said secondary file (20); and
- finally, the sensed fingerprints of the finger of the person to be identified are compared (5) with the fingerprint information stored in said secondary file (20) of only the persons of said selected group.

2. The method as claimed in claim 1, **characterized in that**, after selection (21) of the group of hand shapes substantially identical to those of the person to be identified and before comparing the fingerprints of the finger of the person to be identified with those of the persons of said selected group, an additional selection is performed on the basis of at least one other selection criterion.

3. The method as claimed in claim 2, **characterized in that** the additional selection criterion applied to the persons of the selected hand shape group is a biometric criterion such as sex and/or type.

## Patentansprüche

1. Verfahren zur Identifizierung einer zu einer Bevölkerung gehörenden Person durch Erfassung der Fingerabdrücke mindestens eines Fingers der Hand der Person und Vergleich dieser erfassten Fingerabdrücke mit Fingerabdruckinformationen der Person, die vorher in einer Datei (1) gespeichert wurden, Verfahren, bei dem:
während eines vorhergehenden Schritts der Volkszählung die Form mindestens einer Hand und Fingerabdrücke mindestens eines Fingers der Hand aller Personen der Bevölkerung erfasst werden, und die Handforminformationen in der Datei (1) in Zusammenhang mit mindestens den jeweiligen Fingerabdruckinformationen gespeichert werden, in der Datei (1) eine Sekundärdatei (20) erstellt wird, die Gruppen von im Wesentlichen gleichen Handformen enthält;
dann während eines späteren Schritts der Identifizierung einer zu der Bevölkerung gehörenden Person:
- die Form der Hand der Person gleichzeitig mit der Erfassung (2) der Fingerabdrücke mindestens eines ihrer Finger erfasst wird (3);
- aus der Sekundärdatei (20) eine Gruppe ausgewählt wird (21), die aus den Handformen besteht, die der erfassten Handform der zu identifizierenden Person im Wesentlichen gleich sind; und
- schließlich die erfassten Fingerabdrücke des Fingers der zu identifizierenden Person mit den in der Sekundärdatei (20) gespeicherten Fingerabdruckinformationen nur der Personen der oben genannten ausgewählten Gruppe verglichen werden (5).

2. Verfahren nach Anspruch 1, **dadurch gekennzeichnet, dass** nach der Auswahl (21) der Gruppe der Handformen, die derjenigen der zu identifizierenden Person im Wesentlichen gleich sind, und vor dem Vergleich der Fingerabdrücke des Fingers der zu identifizierenden Person mit denjenigen der Personen der oben genannten ausgewählten Gruppe, eine zusätzliche Auswahl auf der Basis mindestens eines weiteren Auswahlkriteriums durchgeführt wird.

3. Verfahren nach Anspruch 2, **dadurch gekennzeichnet, dass** das an die Personen der ausgewählten Gruppe von Handformen angewendete zusätzliche Auswahlkriterium ein biometrisches Kriterium wie Geschlecht und/oder Typ ist.
